# EUROPEAN PATENT APPLICATION

(11) **EP 4 506 445 A1**
(43) Date of publication of application: **12.02.2025**
(21) Application number: 23803272.6
(22) Date of filing: 29.03.2023
(51) Int. Cl.: C12N 5/074, A23L 13/00, C12N 5/0775

(54) **METHOD FOR COLLECTING STEM CELLS FROM ANIMAL TISSUE**

(30) Priority: 09.05.2022 JP 2022077040
(71) Applicant: Toppan Holdings Inc., Tokyo 110-0016 (JP); OSAKA UNIVERSITY, Suita-shi Osaka 565-0871 (JP)
(72) Inventor: KITANO Shiro, Tokyo 110-0016 (JP); MATSUSAKI Michiya, Suita-shi, Osaka 565-0871 (JP); LOUIS Fiona, Suita-shi, Osaka 565-0871 (JP)
(74) Representative: TBK
(86) International application number: PCT/JP2023/012955
(87) International publication number: WO 2023/218789

(57) **Abstract**

The present invention relates to a method for collecting stem cells from animal tissue, including: a step of bringing at least a surface of the animal tissue into contact with a washing solution, in which the washing solution is a solution containing peracetic acid, hydrogen peroxide, and acetic acid.

## Description

### Technical Field

The present invention relates to a method for collecting stem cells from animal tissue. The present invention also relates to a method for producing cultured meat using stem cells collected through the method.

### Background Art

Cultured meat refers to meat obtained by culturing cells, and is also called "artificial meat," "lab meat," or "clean meat." Natural meat is composed of skeletal muscle cells, adipocytes, fibroblasts, chondrocytes, blood cells, and the like. Therefore, it is necessary for the cells used in the production of cultured meat to have abilities to self-proliferate until they reach a sufficient quantity and to differentiate into the above-described cell types. Stem cells (somatic stem cells) present in animal tissue are promising candidates for such cells (Non-Patent Literature 1).

### Citation List

### Non-Patent Literature

[Non-Patent Literature 1] Reiss J et al., "Cell Sources for Cultivated Meat: Applications and Considerations throughout the Production Workflow" Int. J. Mol. Sci. July 13, 2021; 22(14): 7513

### Summary of Invention

### Technical Problem

Conventionally, antibiotics are used to prevent contamination with various bacteria when collecting stem cells from animal tissue. However, because the use of antibiotics is not able to be applied for edible purposes, stem cells collected through the above-described method for collecting stem cells cannot be used for production of cultured meat. In addition, methods for collecting stem cells from animal tissue without using materials that are not able to be applied for edible purposes are not yet known.

Therefore, an object of the present invention is to provide a method for collecting stem cells that can be used for production of cultured meat from animal tissue. Another object of the present invention is to provide a method for producing cultured meat using stem cells collected through the method.

### Solution to Problem

That is, the present invention relates to, for example, the following inventions.
[1] A method for collecting stem cells from animal tissue, including: a step of bringing at least a surface of the animal tissue into contact with a washing solution, in which the washing solution is a solution containing peracetic acid, hydrogen peroxide, and acetic acid.
[2] The method according to [1], in which the washing solution contains 0.03% v/v to 0.075% v/v peracetic acid, 0.01% v/v to 0.025% v/v hydrogen peroxide, and 0.09% v/v to 0.225% v/v acetic acid.
[3] The method according to [1] or [2], in which the time to bring the animal tissue into contact with the washing solution is 5 minutes to 10 minutes.
[4] The method according to any one of [1] to [3], in which the animal tissue includes adipose tissue.
[5] The method according to any one of [1] to [4], in which the animal tissue includes bovine-derived adipose tissue.
[6] The method according to any one of [1] to [5], in which the stem cells include adipose-derived stem cells.
[7] The method according to any one of [1] to [6], further including: a step of dispersing the animal tissue into a plurality of cell clusters.
[8] The method according to [7], further including: a step of adherently culturing the plurality of cell clusters to remove non-adherent cells; and/or a step of culturing the plurality of cell clusters in a medium for stem cells.
[9] The method according to claim 8, including: performing at least one time of subculture.
[10] The method according to any one of [1] to [9], in which the stem cells are for production of cultured meat.
[11] A method for producing cultured meat, including: a step of carrying out the method according to any one of [1] to [10] to collect stem cells from animal tissue; and a step of culturing the collected stem cells to induce differentiation to construct artificial tissue.

### Advantageous Effects of Invention

According to the present invention, it is possible to provide a method for collecting stem cells that can be used for production of cultured meat from animal tissue. According to the present invention, it is also possible to provide a method for producing cultured meat using stem cells collected through the method.

### Brief Description of Drawings

FIG. 1 shows photographs of adipose-derived stem cells collected from bovine-derived adipose tissue after 2 days and 7 days in culture in Test Example 1.
FIG. 2 shows photographs showing results of lipid staining with Nile red and nuclear staining with Hoechst in artificial tissues obtained by inducing adipose-derived stem cells to differentiate into mature adipocytes in Test Example 2.
FIG. 3 is a graph showing results of an examination of an influence of the number of times of subculture of adipose-derived stem cells on differentiation ability in Test Example 3.

### Description of Embodiments

Hereinafter, embodiments of the present invention will be described in detail. However, the present invention is not limited to the following embodiments.

### [Method for collecting stem cells from animal tissue]

A method for collecting stem cells from animal tissue according to the present embodiment (hereinafter also simply referred to as "collection method") includes a step of bringing at least the surface of animal tissue into contact with a specific washing solution (hereinafter also referred to as "cleaning step"). Here, the washing solution used is a solution containing peracetic acid, hydrogen peroxide, and acetic acid.

The term "stem cells" in the present specification refers to cells that have an ability to self-proliferate and have multipotency. As stem cells, any stem cells (somatic stem cells) present in animal tissue may be sufficient, and specific examples thereof include mesenchymal stem cells (for example, adipose-derived stem cells and bone marrow-derived stem cells), hematopoietic stem cells, muscle stem cells, and neural stem cells. Stem cells are preferably mesenchymal stem cells and more preferably adipose-derived stem cells.

Because the collection method according to the present embodiment includes a process of bringing the surface of animal tissue into contact with a specific washing solution, it is possible to collect stem cells without causing contamination with various bacteria. In addition, the collected stem cells retain an ability to self-proliferate and multipotency. Therefore, since stem cells can be collected without using materials (such as antibiotics) that are not able to be applied for edible purposes, the collected stem cells can be used for production of cultured meat.

In the cleaning step, at least the surface of animal tissue is brought into contact with a specific washing solution. In the cleaning step, a specific washing solution only needs to come into contact with at least the surface of animal tissue, that is, the entire surface of animal tissue, and may come into contact with the interior of animal tissue in addition to the entire surface of the animal tissue. The cleaning step may include a step of bringing at least the surface of animal tissue into contact with a specific washing solution to remove various bacteria on the surface of the animal tissue. The expression "removing various bacteria" refers to sterilizing various bacteria adhering to the surface of collected animal tissue, inhibiting growth of various bacteria adhering to the surface of animal tissue, or physically removing various bacteria from the surface of animal tissue. By bringing at least the surface of animal tissue into contact with a specific washing solution, it becomes possible to collect stem cells without causing contamination with various bacteria. In addition, since the specific washing solution used in the cleaning step does not adversely affect the properties of the stem cells, the collected stem cells maintain their ability to self-proliferate and their multipotency. Moreover, since the specific washing solution used in the cleaning step is able to be applied for edible purposes, the stem cells collected through the collection method according to the present embodiment are able to be applied for production of cultured meat.

The method for bringing animal tissue into contact with a washing solution is not particularly limited as long as at least the surface of animal tissue comes into contact with the washing solution, and examples thereof include a method for immersing animal tissue in a washing solution or a method for spraying or applying a washing solution onto the surface of animal tissue.

Animal tissue is not particularly limited as long as it is animal tissue containing stem cells and may be, for example, derived from mammalian animals such as monkeys, dogs, cats, rabbits, chickens, pigs, cows, horses, mice and rats. Animal tissue is preferably derived from cows, pigs, or chickens, and more preferably derived from cows. The types of animal tissue are not particularly limited, and may include epithelial tissue, muscle tissue, connective tissue, and nerve tissue, and may be tissue derived from epidermal tissue, bone marrow tissue, cartilage tissue, adipose tissue, dental pulp tissue, skeletal muscle tissue, cardiac muscle tissue, and hippocampal tissue. The types of animal tissue are preferably muscle tissue and connective tissue, more preferably skeletal muscle tissue or adipose tissue, and still more preferably adipose tissue. In addition, animal tissue is preferably bovine-derived adipose tissue.

The washing solution in the collection method according to the present embodiment is a solution containing peracetic acid, hydrogen peroxide, and acetic acid. The washing solution according to the present embodiment may contain an aqueous medium in addition to the above-described components. The aqueous medium is not particularly limited, but examples thereof include water and physiological saline such as phosphate-buffered physiological saline (PBS). The washing solution according to the present embodiment can be prepared by diluting a commercially available concentrate (for example, Perasan (registered trademark) (Enviro Tech Chemical Services, Inc., containing 15% v/v peracetic acid, 5% v/v hydrogen peroxide, and 45% v/v acetic acid)) with an aqueous medium, for example. The washing solution according to the present embodiment may further contain components that are able to be applied for edible purposes and may contain, for example, a surfactant, a pH adjuster, and a stabilizer that are able to be applied for edible purposes.

The concentration of peracetic acid in the washing solution according to the present embodiment based on the total amount of washing solution may be 0.012% v/v to 0.15% v/v, 0.022% v/v to 0.12% v/v, or 0.03% v/v to 0.075% v/v.

The concentration of hydrogen peroxide in the washing solution according to the present embodiment based on the total amount of washing solution may be 0.004% v/v to 0.05% v/v, 0.0075% v/v to 0.04% v/v, or 0.01% v/v to 0.025% v/v.

The concentration of acetic acid in the washing solution according to the present embodiment based on the total amount of washing solution may be 0.036% v/v to 0.45% v/v, 0.0675% v/v to 0.36% v/v, or 0.09% v/v to 0.225% v/v.

In the cleaning step, the time over which at least the surface of the above-described animal tissue is brought into contact with the washing solution is not particularly limited, and may be, for example, 1 minute to 20 minutes, 3 minutes to 15 minutes, or 5 minutes to 10 minutes.

The method for collecting stem cells according to the present embodiment may further include one or more steps selected from a step of dispersing animal tissue into a plurality of cell clusters (hereinafter also referred to as "dispersion step"), a step of adherently culturing the dispersed plurality of cell clusters to remove non-adherent cells (hereinafter also referred to as "removal step"), and a step of culturing the dispersed plurality of cell clusters in a medium for stem cells (hereinafter also referred to as "culture step"). By incorporating these steps, the percent yield and yield of stem cells that can be collected from animal tissue improve, and furthermore, the purity of the stem cells in the collected cells increases. Therefore, the percent yield, yield, and purity of the collected stem cells increase, leading to a higher efficiency of differentiation induction into mature cells.

In the dispersion step, animal tissue is dispersed into a plurality of cell clusters. A cell cluster refers to a single cell or a cell aggregation in which a plurality of cells (for example, about 2 to 10 cells) are adhered to each other. The method for dispersing animal tissue into a plurality of cell clusters is not particularly limited, but may be an enzymatic technique or a physical technique. In addition, these techniques may be combined.

As an enzymatic technique, for example, enzymes such as trypsin, collagenase, hyaluronidase, pronase, dispase, and Accutase (registered trademark) may be added, or a chelating agent such as EDTA may be added together with enzymes to be used. Enzymes may be added alone or in combination of two or more thereof. The concentration of enzymes to be added is not particularly limited, but may be 0.001 to 1 mg/mL. In addition, the reaction temperature may be appropriately set according to the optimum temperature of each enzyme, and may be, for example, 30°C to 40°C, and particularly preferably 37°C. The reaction time may be, for example, 10 minutes to 1 hour or may be 20 minutes to 30 minutes.

The physical technique is not particularly limited, and for example, dispersion into a plurality of cell clusters may be performed using an ultrasonic homogenizer, a stirring homogenizer, a high-pressure homogenizer, and the like. In addition, dispersion into more cell clusters can be performed by adjusting the time and frequency of homogenization.

In the removal step, the plurality of cell clusters obtained in the dispersion step are adherently cultured to remove non-adherent cells. The removal step may be a step in which the plurality of cell clusters obtained in the dispersion step are adherently cultured and the culture substrate is washed with an aqueous medium or the like to remove non-adherent cells. Since stem cells adhere to a culture substrate and proliferate, by adherently culturing a plurality of cell clusters to remove non-adherent cells, the purity of stem cells in the dispersed cell clusters can be increased.

The term "adherent culture" refers to culture conducted under the conditions where adhesion occurs through a strong cell-substrate bond between cells and a culture substrate. The culture substrate is not particularly limited, but examples thereof include a flask, a test tube, and a petri dish. In addition, the term "non-adherent cells" refers to cells that do not adhere through the cell-substrate bond as described above and proliferate while floating in a medium. Examples of non-adherent cells include blood cells.

The medium used for adherent culture is not particularly limited, but examples thereof include Dulbecco's Modified Eagle Medium (DMEM) and Iscove's Modified Dulbecco's Medium (IMDM). The medium may be a medium to which serum is added, or may be a serum-free medium. The medium may be a mixed medium obtained by mixing two kinds of media with each other.

The conditions for culturing cells may be any conditions as long as stem cells grow, and suitable culture conditions in the removal step can be set according to the types of cells. For example, the culture temperature may be 20°C to 40°C or may be 30°C to 37°C. The pH of a medium may be 6 to 8 or may be 7.2 to 7.4. The culture time may be 1 to 2 days or may be 3 to 5 days. In addition, after the elapse of a certain period of culture, subculture may be performed. In addition, the term "subculture" refers to an operation of removing a medium from a cell culture system and transferring the cells to a new medium. When removing a medium from a culture system, an operation of detaching cells adhering to a culture substrate from the cell substrate may be further performed. One or more times of subculture are expected to significantly improve the ability to differentiate into mature cells by killing or reducing the number of non-stem cells that may slightly remain in the cell clusters obtained in the dispersion step and by improving the purity of stem cells. Subculture may be performed one or more times, two or more times, three or more times, or four or more times.

In the culture step, the plurality of cell clusters obtained in the dispersion step are cultured in a medium for stem cells. Since the culture step involves culturing the dispersed cell clusters in a medium for stem cells, the yield of stem cells that can be collected from the dispersed cell clusters can be improved. The term "medium for stem cells" refers to a medium in which stem cells can proliferate.

The medium for stem cells is not particularly limited as long as stem cells can proliferate therein, and examples thereof include Dulbecco's Modified Eagle Medium (DMEM) and Iscove's Modified Dulbecco's Medium (IMDM). The medium may be a medium to which serum is added, or may be a serum-free medium. The medium may be a mixed medium obtained by mixing two kinds of media with each other. In addition, the medium for stem cells is preferably a medium in which non-stem cells cannot proliferate. The cell culture conditions may be any conditions under which stem cells proliferate, and may be the same conditions as those in the above-described removal step. In addition, subculture may be performed in the same manner as in the removal step. When performing both the removal step and the culture step, both steps may or may not be carried out simultaneously.

### [Method for producing cultured meat]

A method for producing cultured meat according to the present embodiment includes a step of carrying out the above-described collection method to collect stem cells from animal tissue (hereinafter also referred to as "collection step") and a step of culturing the collected stem cells to induce differentiation to construct an artificial tissue.

The term "artificial tissue" refers to a cell aggregate (bulky cell population) that is artificially created through cell culture, with cells arranged three-dimensionally. When the artificial tissue contains extracellular matrix components to be described below, cells are arranged three-dimensionally via the extracellular matrix components. The shape of the artificial tissue is not particularly limited, and examples thereof include a sheet shape, a spherical shape, an approximately spherical shape, an ellipsoidal shape, an approximately ellipsoidal shape, a hemispherical shape, an approximately hemispherical shape, a semicircular shape, an approximately semicircular shape, a rectangular parallelepiped shape, and an approximately rectangular parallelepiped shape.

The cells of the artificial tissue include at least stem cells obtained through the above-described collection method and cells differentiated from the stem cells. Examples of cells differentiated from stem cells include mesenchymal cells such as fibroblasts, chondrocytes, and osteoblasts, cardiomyocytes, epithelial cells (for example, human gingival epithelial cells), lymphatic endothelial cells, neurons, dendritic cells, hepatocytes, adhesive cells (for example, immune cells), smooth muscle cells (for example, aortic smooth muscle cells (Aorta-SMC)), islet cells, and keratinocytes (for example, human epidermal keratinocytes).

An artificial tissue may have a vascular network between cells. "Having a vascular network between cells" refers to having a structure extending between cells so that branched blood vessels surround the cells, similarly to biological tissue.

The thickness of the above-described artificial tissue is preferably 0.5 mm or more, more preferably 1 mm or more, and still more preferably 2 mm or more. Such an artificial tissue is suitable for cultured meat. The upper limit of the thickness of the artificial tissue is not particularly limited, but may be, for example, 40 mm or less, 30 mm or less, 20 mm or less, 10 mm or less, or 5 mm or less.

Here, the expression "thickness of the artificial tissue" refers to a distance between both ends in a direction perpendicular to the main surface in a case where the artificial tissue has a sheet shape or a rectangular parallelepiped shape. In a case where the above-described main surface is uneven, the thickness refers to a distance therebetween at the thinnest portion of the above-described main surface.

In addition, in a case where the artificial tissue is spherical or substantially spherical, the thickness thereof refers to a diameter thereof. Furthermore, if the artificial tissue is ellipsoidal or substantially ellipsoidal, the thickness thereof refers to a minor axis thereof. In a case where the artificial tissue is substantially spherical or substantially ellipsoidal and its surface is uneven, the thickness thereof refers to the shortest distance between two points where a straight line passing through the gravity center of the artificial tissue and the above-described surface intersect.

Between the above-described collection step and the step of culturing the collected stem cells to induce differentiation to construct an artificial tissue, the collected stem cells may be cultured under the same culture conditions as those in the above-described removal step or the above-described culture step and subcultured after the elapse of a certain period of culture. One or more times of subculture are expected to significantly improve the ability to differentiate into mature cells by killing or reducing the number of non-stem cells that may slightly remain in the stem cells collected in the collection step and by improving the purity of the stem cells. Subculture may be performed one or more times, two or more times, three or more times, or four or more times.

Culturing the stem cells to induce differentiation can be performed through a usual method. When the collected stem cells are adipose-derived stem cells, the adipose-derived stem cells may be cultured in the presence of one or more fatty acids selected from the group consisting of erucic acid, elaidic acid, oleic acid, palmitoleic acid, myristoleic acid, phytanic acid, and pristanic acid. In addition, the culture may be performed in the presence of two or more, three or more, four or more, five or more, or six or more fatty acids selected from the group consisting of erucic acid, elaidic acid, oleic acid, palmitoleic acid, myristoleic acid, phytanic acid, and pristanic acid, and may be performed in the presence of elaidic acid, oleic acid, palmitoleic acid, myristoleic acid, phytanic acid, and pristanic acid. In addition, the culture may be performed in the presence of erucic acid, elaidic acid, oleic acid, palmitoleic acid, myristoleic acid, phytanic acid, and pristanic acid. By performing the culture in the presence of these fatty acids, the differentiation of adipose-derived stem cells into mature adipocytes is further promoted.

A medium is not particularly limited, and a suitable medium can be selected depending on the types of cells to be cultured. The medium may be either a solid medium or a liquid medium, but a liquid medium is preferable. Examples of media include Eagle's MEM medium, DMEM, Modified Eagle medium (MEM), Minimum Essential medium, RPMI, and GlutaMax medium. In addition, a liquid medium may also be used in a gel form such as fibrin gel, hydrogel, Matrigel, collagen gel, or gelatin gel. Cells may be added to a gel-like liquid medium, or a liquid medium containing cells may be gelled and used. The medium may be a medium to which serum is added, or may be a serum-free medium. The medium may be a mixed medium obtained by mixing two kinds of media with each other.

Cells of an artificial tissue are cells that include at least adipose-derived stem cells collected through the above-described collection method. When the cells are cultured to induce differentiation in a medium in which the above-described fatty acids are contained, the content of each fatty acid in the medium may be, for example, 0.1 µM to 200 µM, 2 µM to 100 µM, 10 µM to 60 µM, 30 µM to 50 µM, 30 µM to 150 µM, or 80 µM to 120, may be 1 µM or more, 5 µM or more, 10 µM or more, 20 µM or more, 30 µM or more, 40 µM or more, 50 µM or more, 60 µM or more, 70 µM or more, 80 µM or more, or 90 µM or more, and may be 200 µM or less, 150 µM or less, 120 µM or less, 100 µM or less, 80 µM or less, or 70 µM or less.

Each fatty acid may be, for example, 2.0×10⁻¹¹mol to 4.0×10⁻⁸mol, or 1.0×10⁻⁹mo1 to 2.0×10⁻⁸mol per 1×10⁶ cells of adipose-derived stem cells. The weight of each fatty acid may be, for example, 6 ng to 15 µg, or 250 ng to 10 µg per 1×10⁶ cells of adipose-derived stem cells.

The culture to induce differentiation in not particularly limited and can be performed under suitable conditions according to the types of cells to be cultured. For example, the temperature for culture to induce differentiation may be 20°C to 40°C or may be 30°C to 37°C. The pH of a medium may be 6 to 8 or may be 7.2 to 7.4. The time for culture to induce differentiation may be 24 hours to 336 hours, 72 hours to 336 hours, 96 hours to 384 hours, or 96 hours to 288 hours.

An incubator (support) used for culturing cells of an artificial tissue is not particularly limited and may be, for example, a well insert, a low adhesion plate, or a plate with a bottom surface shape such as a U-shape or a V-shape. The above-described cells may be cultured while being adhered to a support, may be cultured without being adhered to a support, or may be cultured while being separated from a support in the middle of culture. In a case where the above-described cells are cultured without being adhered to a support or in a case where the above-described cells are cultured while being separated from a support in the middle of culture, a plate having a bottom surface shape such as a U-shape or a V-shape that prevents the above-described cells from adhering to a support or a low adhesion plate is preferably used.

Induction of differentiation of stem cells in the step of culturing the collected stem cells to induce differentiation to construct an artificial tissue may be performed by culturing cells including at least the stem cells collected through the above-described collection method in the presence of fragmented extracellular matrix components. By performing culture with fragmented extracellular matrix components, it is possible to obtain an artificial tissue in which the cells are three-dimensionally arranged via the fragmented extracellular matrix components. The artificial tissue is preferably one in which the fragmented extracellular matrix components are arranged in gaps between the above-described cells. Cells in the cell-cell mixture may be of the same type or of different types.

Fragmented extracellular matrix components can be obtained by fragmenting extracellular matrix components. The "extracellular matrix components" in the present specification are extracellular matrix molecule aggregates formed by a plurality of extracellular matrix molecules. The extracellular matrix means a substance existing outside cells in an organism. An arbitrary substance can be used as the extracellular matrix as long as this does not adversely affect growth of cells and formation of cell aggregates. Specific examples thereof include collagen, elastin, proteoglycans, fibronectin, hyaluronic acid, laminin, vitronectin, tenascin, entactin, and fibrillin, but the present invention is not limited thereto. These extracellular matrix components may be used alone or in combination. For example, extracellular matrix components may contain collagen components or may be collagen components. The extracellular matrix components in the present embodiment are preferably substances existing outside animal cells, that is, animal extracellular matrix components.

Examples of collagen include fibrous collagen and non-fibrous collagen. Fibrous collagen means collagen that is a main component of collagen fibers, and specific examples thereof include type I collagen, type II collagen, and type III collagen. Examples of non-fibrous collagen include type IV collagen.

Examples of proteoglycans include, but are not limited to, chondroitin sulfate proteoglycans, heparan sulfate proteoglycans, keratan sulfate proteoglycans, and dermatan sulfate proteoglycans.

An extracellular matrix component may contain at least one selected from the group consisting of collagen, laminin, and fibronectin and preferably contains collagen. Collagen is preferably fibrous collagen and more preferably type I collagen. Commercially available collagen may be used as fibrous collagen, and specific examples thereof include porcine skin-derived type I collagen manufactured by NH Foods Ltd.

The "fragmentation" means that an aggregate of extracellular matrix molecules is made smaller in size. The fragmentation may be performed under the conditions of cleaving the bond within extracellular matrix molecules or may be performed under the conditions of not cleaving the bond within extracellular matrix molecules. Unlike enzymatic treatment, an extracellular matrix fragmented through application of physical force usually does not change in molecular structure from before the fragmentation (the molecular structure is maintained). The fragmented extracellular matrix components may include defibrated extracellular matrix components in which the above-described extracellular matrix component is defibrated through application of physical force. Defibration is an aspect of fragmentation and is performed under the conditions of, for example, not cleaving the bond within extracellular matrix molecules.

The method for fragmenting extracellular matrix components is not particularly limited. As the method for defibrating extracellular matrix components, extracellular matrix components may be defibrated through application of physical force with an ultrasonic homogenizer, a stirring homogenizer, a high-pressure homogenizer, and the like, for example. In a case of using a stirring homogenizer, extracellular matrix components may be homogenized as they are or may be homogenized in an aqueous medium such as physiological saline or in an organic solvent such as ethanol. In addition, millimeter-sized and nanometer-sized defibrated extracellular matrix components can also be obtained by adjusting, for example, the time and the number of times of homogenizing. Defibrated extracellular matrix components can also be obtained through defibration by repeating freezing and thawing.

Fragmented extracellular matrix components may include at least some defibrated extracellular matrix components. In addition, fragmented extracellular matrix components may consist of only defibrated extracellular matrix components. In other words, fragmented extracellular matrix components may be defibrated extracellular matrix components. Defibrated extracellular matrix components preferably include defibrated collagen components. Defibrated collagen components preferably maintain a triple helix structure derived from collagen. Defibrated collagen components may be components partially maintaining a triple helix structure derived from collagen.

For example, fragmented extracellular matrix components may include fragmented collagen components and may consist of fragmented collagen components.

At least some fragmented extracellular matrix components may be cross-linked between or within molecules. Extracellular matrix components may be cross-linked within or between extracellular matrix molecules constituting the extracellular matrix components.

The content of extracellular matrix components in an artificial tissue based on (the dry weight of) the artificial tissue may be 0.01 to 90 mass%, preferably 10 to 90 mass%, 10 to 80 mass%, 10 to 70 mass%, 10 to 60 mass%, 1 to 50 mass%, and 10 to 50 mass%, and more preferably 10 to 30 mass% and 20 to 30 mass%.

A residual rate of an artificial tissue subjected to trypsin treatment at a trypsin concentration of 0.25%, a temperature of 37°C, a pH of 7.4, and a reaction time of 15 minutes is preferably 70% or more, more preferably 80% or more, and still more preferably 90% or more. Such an artificial tissue is stable because it is unlikely to be decomposed by an enzyme during or after culture. The above-described residual rate can be calculated from the mass of an artificial tissue before and after trypsin treatment, for example.

The residual rate of the above-described artificial tissue subjected to collagenase treatment at a collagenase concentration of 0.25%, a temperature of 37°C, a pH of 7.4, and a reaction time of 15 minutes may be greater than or equal to 70%, more preferably greater than or equal to 80%, and still more preferably greater than or equal to 90%. Such an artificial tissue is stable because it is unlikely to be decomposed by an enzyme during or after culture.

One embodiment of a method for producing an artificial tissue may include incorporating fibrinogen and thrombin simultaneously or separately. By incorporating fibrinogen and thrombin, they react to form fibrin. The content of fibrinogen based on a medium may be, for example, 1 to 10 mg/mL, 2 to 8 mg/mL, or 5 to 6 mg/mL.

### [Examples]

Hereinafter, the present invention will be described more specifically based on the examples. However, the present invention is not limited thereto.

### [Test Example 1: Collection of adipose-derived stem cells from bovine-derived adipose tissue and culture of adipose-derived stem cells]

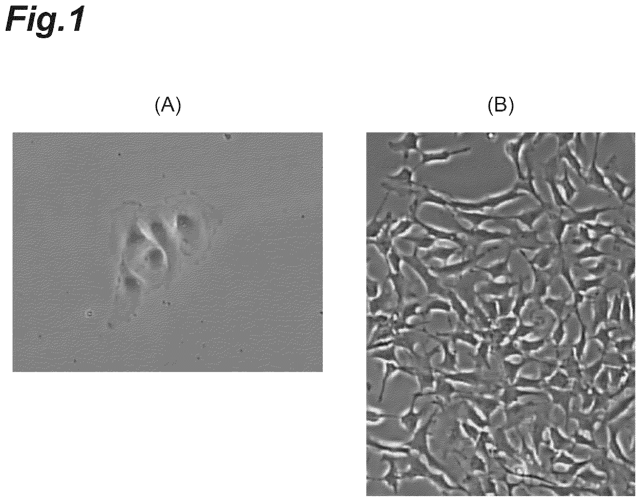

Adipose-derived stem cells were collected from bovine-derived adipose tissue according to the following procedure. Perasan (registered trademark) (manufactured by Enviro Tech Chemical Services, Inc., containing 15% v/v peracetic acid, 5% v/v hydrogen peroxide, and 45% v/v acetic acid) was diluted with phosphate-buffered physiological saline (PBS) to 0.2% v/v to prepare a washing solution containing 0.03% v/v peracetic acid, 0.01% v/v hydrogen peroxide, and 0.09% v/v acetic acid. The bovine-derived adipose tissue was immersed in the above-described washing solution and washed for 5 minutes. Furthermore, the bovine-derived adipose tissue was washed three times with PBS for five minutes. Thereafter, visible blood vessels in the bovine-derived adipose tissue were removed, and the bovine-derived adipose tissue was finely chopped with sterilized forceps and scissors to create small tissue pieces about 3 mm in size. These bovine-derived adipose tissue pieces were transferred to a 6-well plate at 2 g per well. 3 mL of 0.1% trypsin was added to the wells, bovine-derived adipose tissue pieces were incubated at 280 rpm and 37°C for 30 minutes and dispersed into cell clusters. Thereafter, 5 mL of DMEM containing 10% fetal bovine serum (FBS) was added to the wells to stop the reaction of trypsin, and the dispersed bovine-derived adipose tissue pieces were further dispersed through pipetting. The dispersed cells were centrifuged at 1,000 rpm for 10 minutes to precipitate them, and the precipitated cells were suspended in DMEM containing 10% FBS. This cell suspension was filtered through a cell strainer with a mesh size of 40 µm and then seeded in a 10 cm dish and immersed in DMEM containing 10% FBS for 3 days. Three days later, a 10 cm dish with adipose-derived stem cells attached was washed five times with 5 mL of PBS to obtain adipose-derived stem cells. The collected adipose-derived stem cells were subjected to medium exchange using DMEM every 2 to 3 days until 70% confluency was reached. Photographs after 2 days and 7 days in culture are sequentially shown in FIG. 1(A) and 1(B).

By washing the bovine-derived adipose tissue with a solution (washing solution) containing peracetic acid, hydrogen peroxide, and acetic acid, adipose-derived stem cells capable of proliferating without contamination could be collected from the bovine-derived adipose tissue (FIGS. 1(A) and 1(B)).

### [Test Example 2: Construction of artificial tissue]

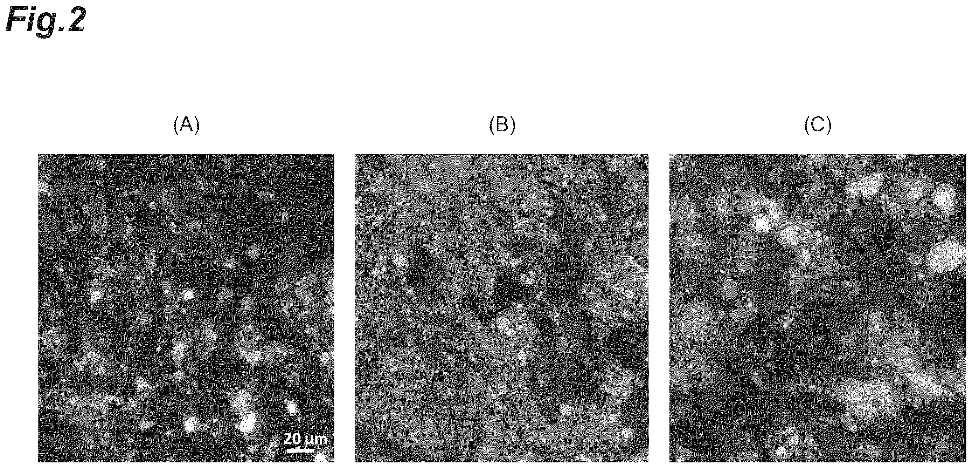

A dispersion containing collagen components (CMF) defibrated through a usual method was obtained from porcine skin-derived collagen type I sponge fragments (manufactured by NH Foods Ltd.). Defibrated collagen components (CMF) were obtained as dried products by freeze-drying the dispersion through a usual method. At the time of use, the fragmented collagen was dispersed into a medium (DMEM) containing serum to a concentration of 10 mg/mL. 2 µL of FBS-free DMEM containing 1.2 weight% (final concentration) defibrated collagen components described above, 6 mg/mL (final concentration) fibrinogen, 3 U/mL (final concentration) thrombin, and the 5×10⁶ cells/mL adipose-derived stem cells collected in Test Example 1 were seeded onto a 96-well plate (manufactured by Iwaki) and incubated for 15 minutes, and then, 200 µL of DMEM was added thereto to perform culture for 48 hours. Thereafter, medium exchange was performed using 200 µL of DMEM containing 100 µm each of erucic acid, elaidic acid, oleic acid, palmitoleic acid, myristoleic acid, phytanic acid, and pristanic acid, the medium was replaced every 2 days for culture to induce differentiation of the adipose-derived stem cells into mature adipocytes to obtain an artificial tissue. Photographs obtained by lipid-staining the artificial tissue on day 3 of differentiation (day 5 after seeding), day 7 of differentiation (day 9 after seeding), and day 14 of differentiation (day 16 after seeding) with Nile red, performing nuclear staining through Hoechst staining, and performing fluorescence observation using a Confocal Quantitative Image Cytometer CQ (Yokogawa Electric Corporation) are sequentially shown in FIGS. 2(A) to 2(C).

On day 14 of differentiation, cells with large lipid droplets were observed in the artificial tissue (FIG. 2(C)), and it was found that the adipose-derived stem cells collected through the technique in Test Example 1 had differentiated into mature adipocytes. Therefore, it was shown that the adipose-derived stem cells could be collected from animal tissue without contamination through the technique of Test Example 1 and that these stem cells not only have the ability to proliferate but also maintain the ability to differentiate into mature adipocytes. In addition, it was shown that cultured meat could also be produced using the stem cells collected through the method.

### [Test Example 3: Confirmation of influence of number of times of subculture of collected adipose-derived stem cells on differentiation ability]

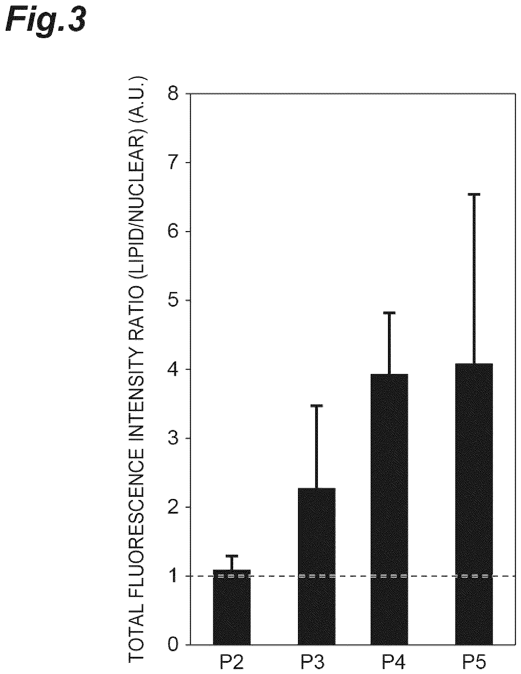

The adipose-derived stem cells collected through the method described in Test Example 1 were cultured in IMEM medium containing 10% bovine serum, and adipose-derived stem cell samples were prepared which had been subjected to subculture once, twice, three times, and four times (referred to as P2, P3, P4, and P5, respectively). Using each sample, artificial tissues were constructed through the same method as in Test Example 2, followed by lipid and nuclear staining in the same manner, and fluorescence observation was performed. Using image analysis software (ImageJ, National Institutes of Health), the total fluorescence intensity of lipid staining (Nile red) and nuclear staining (Hoechst) was quantified, and the fluorescence intensity of lipid staining (Nile red) relative to the fluorescence intensity of nuclear staining (Hoechst) was calculated. The results thereof are shown in FIG. 3. The vertical axis in FIG. 3 shows the ratio of the fluorescence intensity of lipid staining (Nile red) to the fluorescence intensity of nuclear staining (Hoechst) calculated for each sample, the ratio being standardized by the fluorescence intensity ratio in tissue cultured under undifferentiated conditions.

By increasing the number of times of subculture, the above-described fluorescence intensity ratio was maximized after P4. Normally, cells that have not undergone cancerization (immortalization) gradually change their traits with successive subculture, and it is not expected to have a favorable influence on subsequent functions. However, according to the results of Test Example 3, it was surprisingly found that increasing the number of times of subculture improves the differentiation ability of the adipose-derived stem cells. The exact reason is unclear, but it is possible that other cell types that remained slightly within the cell population after the collection step of the adipose-derived stem cells had an unexpected adverse effect on differentiation into adipocytes, and that the death of these cell types through successive subculture improved the purity of the stem cells and the differentiation efficiency.

## Claims

1. A method for collecting stem cells from animal tissue, comprising:
a step of bringing at least a surface of the animal tissue into contact with a washing solution,
wherein the washing solution is a solution comprising peracetic acid, hydrogen peroxide, and acetic acid.

2. The method according to claim 1,
wherein the washing solution comprises 0.03% v/v to 0.075% v/v peracetic acid, 0.01% v/v to 0.025% v/v hydrogen peroxide, and 0.09% v/v to 0.225% v/v acetic acid.

3. The method according to claim 1,
wherein the time over which the animal tissue is brought into contact with the washing solution is 5 minutes to 10 minutes.

4. The method according to claim 1,
wherein the animal tissue comprises adipose tissue.

5. The method according to claim 1,
wherein the animal tissue comprises bovine-derived adipose tissue.

6. The method according to claim 1,
wherein the stem cells comprise adipose-derived stem cells.

7. The method according to claim 1, further comprising:
a step of dispersing the animal tissue into a plurality of cell clusters.

8. The method according to claim 7, further comprising:
a step of adherently culturing the plurality of cell clusters to remove non-adherent cells; and/or
a step of culturing the plurality of cell clusters in a medium for stem cells.

9. The method according to claim 8, comprising:
performing subculture at least once.

10. The method according to claim 1,
wherein the stem cells are for production of cultured meat.

11. A method for producing cultured meat, comprising:
a step of carrying out the method according to any one of claims 1 to 10 to collect stem cells from animal tissue; and
a step of culturing the collected stem cells to induce differentiation to construct an artificial tissue.
